# EUROPEAN PATENT SPECIFICATION

(11) **EP 3 340 836 B1**
(45) Date of publication and mention of the grant of the patent: **01.07.2020**
(21) Application number: 16759719.4
(22) Date of filing: 24.08.2016
(51) Int. Cl.: A47B 95/00, A47B 96/06

(54) **IMPROVEMENTS IN MOUNTING DEVICES**
VERBESSERUNGEN IN MONTAGEVORRICHTUNGEN
AMÉLIORATIONS APPORTÉES À DES DISPOSITIFS D'ASSEMBLAGE

(30) Priority: 26.08.2015 GB 201515162
(43) Date of publication of application: 04.07.2018
(73) Proprietor: Titus d.o.o. Dekani, 6271 Dekani (SI)
(72) Inventor: SVARA, Valter, 6310 Izola (SI); MIGLI, Carlo, 23900 Lecco LC (IT)
(74) Representative: Treeby, Philip David William
(86) International application number: PCT/EP2016/070007
(87) International publication number: WO 2017/032812

(56) References cited:
- EP-A1- 3 111 802
- CN-U- 202 234 015
- DE-U1- 9 201 572
- US-A1- 2010 219 144
- US-A1- 2011 174 939

## Description

This invention relates to mounting devices, and more particularly, though not exclusively, to devices for mounting furniture units such as cabinets on walls.

US 2010/219144 A and US 2011/174939 are hereby acknowledged.

According to the present invention there is provided a wall mounting device for mounting a unit on a wall, the device comprising a bracket assembly for attachment to the unit, and a support for anchoring to the wall, the bracket assembly comprising hooking means for engaging the bracket assembly on the support, in use, for suspending said unit therefrom, wherein said hooking means comprises a releasable locking mechanism movable into a locking position for blocking disengagement of the bracket assembly from the support, with said locking mechanism incorporating an upwardly facing latching surface and the support incorporating a downwardly facing ledge that is engagable therewith to prevent the bracket assembly from being lifted off the support after engagement therewith, with the locking mechanism being arranged to move into its locking position automatically upon engagement of the bracket assembly with the support, wherein said latching surface is provided on a separate member that is mounted on the bracket assembly for movement relative thereto,
wherein the member is in the form of a lever that is pivotally mounted on the bracket assembly,
wherein the lever is pivotable between a locking position in which it prevents disengagement of the bracket assembly from the support and an unlocked position in which it allows disengagement of the bracket assembly from the support, and characterised in further comprising releasable retainer means for holding the lever in its unlocked position.

Preferred aspects are set out in the dependent claims.

By way of example, an embodiment of the invention will now be described with reference to the accompanying drawings, in which:
Figure 1 shows a wall mounting device according to the invention,
Figure 2 is a detail view of the locking lever of the device of Figure 1,
Figures 3a, 3b and 3c show the device of Figure 1 in use with a furniture unit, and
Figure 4 is an enlarged detail view of the locking assembly.

Wall hanging brackets for mounting furniture units such as cabinets and the like on walls are generally known. These typically involve brackets for attachment to the unit to engage a support affixed to the wall. These brackets commonly include mechanisms for making horizontal and vertical adjustments to enable the position and/or inclination of the unit to be adjusted once mounted. Typically, however, conventional brackets do not have any means of preventing their accidental dislodgement.

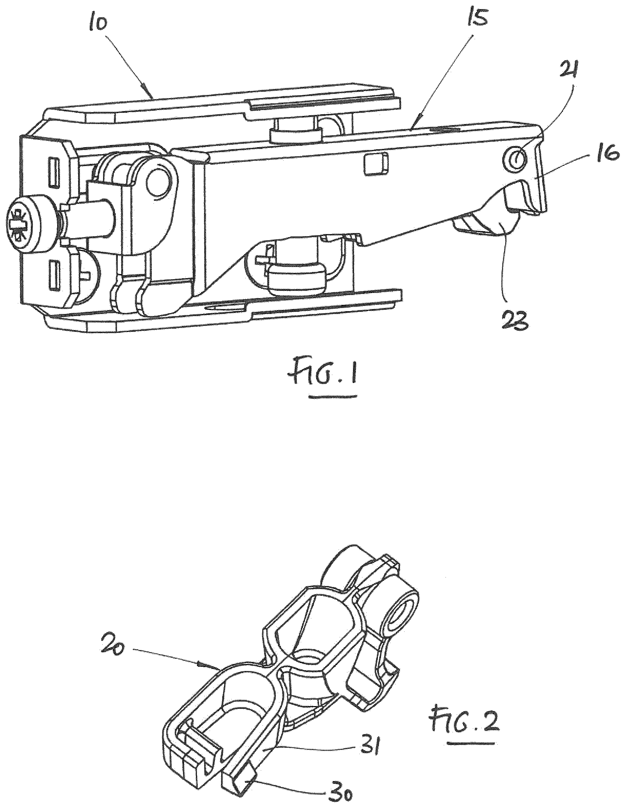

The device 10 seen in Figure 1 is in the form of a bracket assembly for mounting a furniture unit such as a cabinet or the like on a wall. The unit will typically be mounted using two of these devices 10, attached to the back of the unit at each top corner. The device 10 incoporates horizontal and vertical adjusting mechanisms to allow for adjustment of the unit after mounting. Normally, the device 10 will be hidden by a decorative cover, but this has been omitted from the drawings for the sake of clarity.

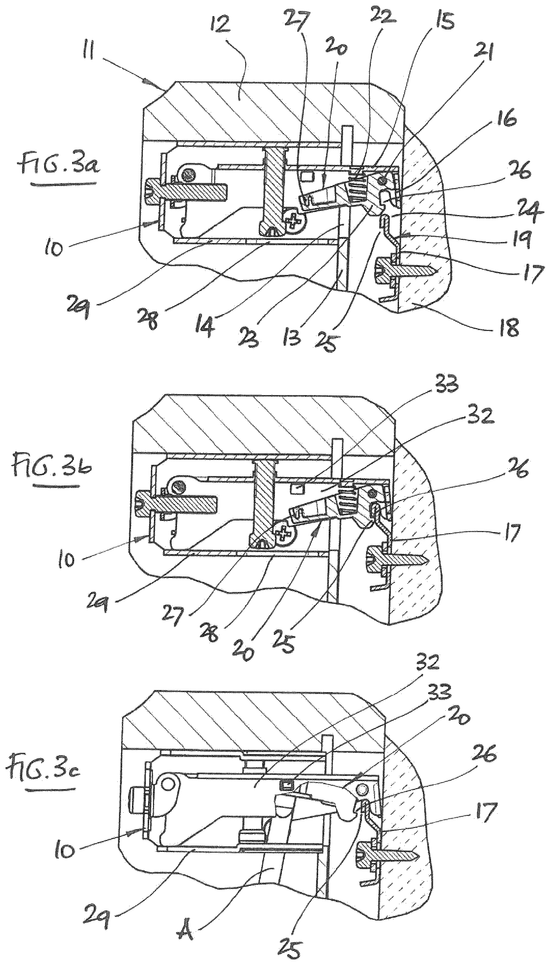

The components of the device 10 and its manner of operation are seen in Figures 3a, 3b and 3c. The device 10 is secured to the inside of the unit 11 at the junction of its top frame 12 and backboard 13 by suitable means such as screws. The device 10 is designed to partly protrude out of the back of the unit 11 through a hole 14 in the backboard 13. The protruding part of the device 10 comprises a rigid arm 15. The arm 15 extends generally horizontally out of the unit 11 and ends in a downwardly extending hook 16. The hook 16 is designed to engage a rigid support 17 which is anchored to a wall 18 by suitable means such as screws. Here, the support 17 is in the form of a horizontally arranged elongate rail.

The support 17, seen in Figures 3a, 3b and 3c in cross-section, has a lip 19 that extends upwardly and outwardly from the surface of the wall 18. The gap thereby created is designed to receive the hook 16 of the arm 15 as the unit 11 is lowered into position. The support 17 is thereby able to take the weight of the unit 11 and its contents.

The device 10 here has a safety locking mechanism to ensure that the unit 11 cannot become accidentally dislodged from its mounting on the wall 18 by being inadvertently lifted off the support 17. The locking mechanism comprises a lever 20 pivotally mounted on the arm 15 to enagage and latch onto the support 17 in use. The lever 20 is mounted on the arm 15 by a pivot pin 21 and spring-loaded by a compression spring 22. The biassing action of spring 22 is arranged to urge the lever 20 towards its locking position as seen in Figure 3b.

The lever 20 has a nose section 23. The free edge of the lip 19 of the support 17 is doubled over, as seen in Figures 3a, 3b and 3c, to create a rounded profile 24. In use, the lever 20 comes into contact with the support 17 by engagement of the nose section 23 with the rounded profile 24. This is the point illustrated in Figure 3a. The contouring of the rounded profile 24 in this manner helps to reduce frictional forces involved in the interaction between the lever 20 and the support 17, as described below, and the nose section 23 of the lever 20 is preferably also contoured for similar reasons.

The doubling over of the free edge of the lip 19 of the support 17 creates a downwardly facing ledge 25, as seen in Figures 3a, 3b and 3c. The nose section 23 of the lever 20 is rebated to form an upwardly facing latching surface 26. It will be seen that further lowering of the unit 11 from the position seen in Figure 3a will result in the nose section 23 riding over the rounded profile 24 of the support 17, causing the lever 20 to pivot as it does so, against the biassing action of the spring 22. Eventually, the latching surface 26 of the lever 20 will be sprung under the ledge 25 of the support 17 under the biassing action of the spring 22. This is the position seen in Figure 3b. This is the locking position of the locking mechanism. In this position, it will be seen that the ledge 25 constitutes a physical block against possible upward movement of the latching surface 26, hence positively preventing the possibility of the device 10 moving out of its engagement with the support 17.

It will be noted that the lever 20 is designed to move automatically into its locking position upon lowering of the device 10 onto the support 17. This means that when a unit incorporating the device 10 is lowered into position on a support 17, the locking mechanism will be activated without need of intervention by hand or by tool. This contrasts with many conventional systems, which require a manual step in the mounting process.

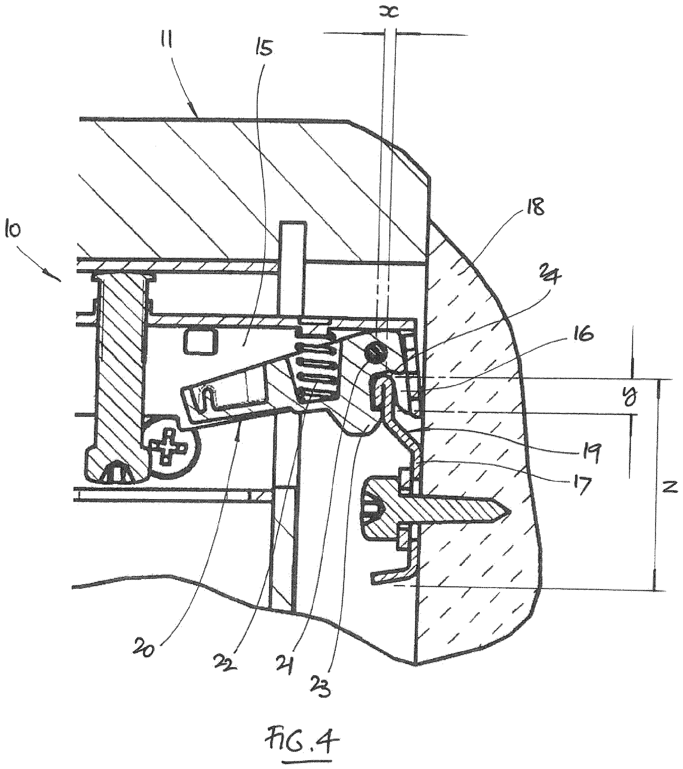

It is to be noted that the geometry of the lever 20 and its manner of mounting are chosen so as to ensure that it will automatically tend to fall towards its locking position as seen in Figure 3b under force of gravity. This arrangement means that the locking function of the device 10 is effectively fail safe, because the lever 20 will still tend to perform its proper role, even if the spring 22 goes missing or otherwise ceases to function. The fail safe functionality of the lever 20 is further assisted by arranging for the axis of the pin 21 that pivotally mounts it to be slightly offset from the point of contact between the rigid arm 16 and the support 17. This offset is indicated by measurement x in Figure 4.

It is also to be noted that the vertical height of the doubled over lip 19 of the support 17, indicated by measurement y in Figure 4, is considerably less than the overall height of the support 17 itself, indicated by measurement z in Figure 4. In this case, measurement y is less than a fifth of measuremnt z. Locking mechanisms for conventional wall mounting brackets typically span between both upper and lower edges of the supporting rail. The arrangement seen here thus offers a significantly more compact solution that needs far less space than conventional devices.

The device 10 is provided with a release mechanism for unlocking the locking mechanism. The free end 27 of the lever 20 opposite to its nose section 23 is accessible via a hole 28 in a frame section 29 of the device 10. This enables the lever 20 to be pivoted by pressing with a tool such as a screwdriver A. This is the situation seen in Figure 3c. It will be seen that pressing on the free end 27 of the lever 20 pivots its latching surface 26 out of engagement with the ledge 25, against the biasing action of the spring 22, thus allowing the unit 11 to be lifted upwardly and off the support 17. This is the unlocked position of the locking mechanism.

The lever 20 conveniently has an automatic retaining mechanism to hold it in its unlocked position. This is seen in Figure 2. Here, the retaining mechanism is in the form of a keeper 30 formed on a resiliently flexible wing 31 of the lever 20. The wing 31 is designed to resiliently deflect as it engages another frame section 32 of the device 10, as the lever 20 is pivoted towards its unlocked position, until the keeper 30 is able to spring into a hole 33. The engagement of the keeper 30 in the hole 33 will then retain the lever 20 in its unlocked position, allowing for removal of the unit 11. This is the position seen in Figure 3c. The lever 20 can be allowed to spring back to its start position under the biasing action of the spring 22 simply by pressing the keeper 30 out of its engagement with the hole 33.

## Claims

1. A wall mounting device for mounting a unit (11) on a wall (18), the device comprising a bracket assembly (10) for attachment to the unit, and a support (17) for anchoring to the wall, the bracket assembly comprising hooking means (16) for engaging the bracket assembly on the support, in use, for suspending said unit therefrom, wherein said hooking means comprise a releasable locking mechanism (20) movable into a locking position for blocking disengagement of the bracket assembly from the support, with said locking mechanism incorporating an upwardly facing latching surface (26) and the support incorporating a downwardly facing ledge (25) that is engagable therewith to prevent the bracket assembly from being lifted off the support after engagement therewith, with the locking mechanism being arranged to move into its locking position automatically upon engagement of the bracket assembly with the support, wherein said latching surface (26) is provided on a separate member (20) that is mounted on the bracket assembly (10) for movement relative thereto,
wherein the member (20) is in the form of a lever (20) that is pivotally mounted on the bracket assembly (10),
wherein the lever (20) is pivotable between a locking position in which it prevents disengagement of the bracket assembly (10) from the support (17) and an unlocked position in which it allows disengagement of the bracket assembly from the support, and **characterised in** further comprising releasable retainer means (30, 31) for holding the lever (20) in its unlocked position.

2. A device as claimed in claim 1 wherein the bracket assembly is engaged on the support by being lowered onto it.

3. A device as claimed in claim 1 and further including means for biassing the lever (20) towards its locking position.

4. A device as claimed in claim 3 wherein said biassing means includes a spring (22).

5. A device as claimed in claim 3 or claim 4 wherein said biassing means is assisted by the force of gravity.

6. A device as claimed in any preceding claim wherein the support (17) is in the form of an elongate rail.

7. A device as claimed in claim 6 wherein the rail (17) has a lip (19) that extends upwardly and outwardly from the wall (18).

8. A device as claimed in claim 7 where the free edge of the lip (19) is turned over to form a curved profile (24).

9. A device as claimed in claim 8 wherein the locking mechanism (20) is arranged to come into contact with the support (17) via said curved profile (24).

10. A device as claimed in claim 8 or claim 9 wherein the turned over free edge of the lip (19) forms said downwardly facing ledge (25).

11. A device as claimed in any one of claims 8 to 10 wherein the vertical height of the turned over free edge of the lip (19) is less than the overall height of the support (17).

12. A device as claimed in any one of claims 1 to 11 wherein the axis of the pivotal mount for the lever (20) is offset from the point of contact between the bracket assembly (10) and the support (17).

## Patentansprüche

1. Wandmontagevorrichtung zum Montieren einer Einheit (11) an einer Wand (18), wobei die Vorrichtung eine Halterungsanordnung (10) zum Anbringen an der Einheit und einen Träger (17) zum Verankern an der Wand umfasst, wobei die Halterungsanordnung Hakenmittel (16) für den Eingriff der Halterungsanordnung mit dem Träger während des Gebrauchs umfasst, um die Einheit davon abzuhängen, wobei die Hakenmittel einen lösbaren Arretierungsmechanismus (20) umfassen, der in eine Arretierungsposition beweglich ist, um das Ausklinken der Halterungsanordnung von dem Träger zu sperren, wobei der Arretierungsmechanismus eine nach oben weisende Verriegelungsfläche (26) beinhaltet und der Träger eine nach unten weisende Leiste (25) umfasst, die damit in Eingriff gebracht werden kann, um zu verhindern, dass die Halterungsanordnung nach ihrem Eingriff mit dem Träger von diesem abgehoben wird, wobei der Arretierungsmechanismus dafür angeordnet ist, sich auf den Eingriff der Halterungsanordnung mit dem Träger hin automatisch in seine Arretierungsposition zu bewegen, wobei die Verriegelungsfläche (26) an einem separaten Element (20) bereitgestellt ist, das für die Bewegung im Verhältnis zur Halterungsanordnung (10) an selbiger montiert ist,
wobei das Element (20) in Form eines Hebels (20) vorliegt, der schwenkbar an die Halterungsanordnung (10) montiert ist,
wobei der Hebel (20) zwischen einer Arretierungsposition, in der er das Lösen der Halterungsanordnung (10) von dem Träger (17) verhindert, und einer entsperrten Position, in der er das Ausklinken der Halterungsanordnung vom Träger gestattet, schwenkbar ist und
**dadurch gekennzeichnet, dass** sie ferner lösbare Haltemittel (30, 31) zum Halten des Hebels (20) in seiner entriegelten Position umfasst.

2. Vorrichtung nach Anspruch 1, wobei die Halterungsanordnung mit dem Träger in Eingriff gebracht wird, indem sie auf ihn abgesenkt wird.

3. Vorrichtung nach Anspruch 1 und ferner Mittel zum Vorspannen des Hebels (20) in seine Arretierungsposition beinhaltend.

4. Vorrichtung nach Anspruch 3, wobei die Vorspannmittel eine Feder (22) beinhalten.

5. Vorrichtung nach Anspruch 3 oder Anspruch 4, wobei die Vorspannmittel von der Schwerkraft unterstützt werden.

6. Vorrichtung nach einem der vorhergehenden Ansprüche, wobei der Träger (17) in Form einer länglichen Schiene vorliegt.

7. Vorrichtung nach Anspruch 6, wobei die Schiene (17) eine Lippe (19) aufweist, die sich von der Wand (18) nach außen und nach oben erstreckt.

8. Vorrichtung nach Anspruch 7, wobei die freie Kante der Lippe (19) umgebogen ist, um ein gekrümmtes Profil (24) zu bilden.

9. Vorrichtung nach Anspruch 8, wobei der Arretierungsmechanismus (20) derart angeordnet ist, dass er mittels des gekrümmten Profils (24) in Kontakt mit dem Träger (17) gelangt.

10. Vorrichtung nach Anspruch 8 oder 9, wobei der umgebogene freie Rand der Lippe (19) die genannte nach unten weisende Leiste (25) bildet.

11. Vorrichtung nach einem der Ansprüche 8 bis 10, wobei die vertikale Höhe der umgebogenen freien Kante der Lippe (19) kleiner als die Gesamthöhe des Trägers (17) ist.

12. Vorrichtung nach einem der Ansprüche 1 bis 11, wobei die Achse des Schwenkhalters für den Hebel (20) zu dem Punkt des Kontakts zwischen der Halterungsanordnung (10) und dem Träger (17) versetzt ist.

## Revendications

1. Dispositif de montage mural servant à monter un élément (11) sur un mur (18), le dispositif comportant un ensemble formant applique (10) à des fins de fixation sur l'élément, et un support (17) servant à des fins d'ancrage sur le mur, l'ensemble formant applique comportant des moyens formant crochet (16) servant à mettre en prise l'ensemble formant applique sur le support, lors de l'utilisation, à des fins de suspension dudit élément en provenance de celui-ci, dans lequel lesdits moyens formant crochet comportent un mécanisme de verrouillage libérable (20) mobile jusque sur une position de verrouillage pour bloquer toute mise hors prise de l'ensemble formant applique par rapport au support, ledit mécanisme de verrouillage incorporant une surface d'enclenchement orientée vers le haut (26) et le support incorporant un rebord orienté vers le bas (25) qui est en mesure de se mettre en prise avec celui-ci pour empêcher l'ensemble formant applique d'être soulevé du support après la mise en prise avec celui-ci, le mécanisme de verrouillage étant agencé pour se déplacer jusque dans sa position de verrouillage automatiquement lors de la mise en prise de l'ensemble formant applique avec le support, dans lequel ladite surface d'enclenchement (26) est mise en œuvre sur un organe séparé (20) qui est monté sur l'ensemble formant applique (10) à des fins de mouvement par rapport à celui-ci,
dans lequel l'organe (20) est sous la forme d'un levier (20) qui est monté de manière pivotante sur l'ensemble formant applique (10),
dans lequel le levier (20) est en mesure de pivoter entre une position de verrouillage dans laquelle il empêche toute mise hors prise de l'ensemble formant applique (10) par rapport au support (17) et une position déverrouillée dans laquelle il permet toute mise hors prise de l'ensemble formant applique par rapport au support, et **caractérisé en ce qu'**il comporte par ailleurs des moyens de retenue libérables (30, 31) servant à retenir le levier (20) dans sa position déverrouillée.

2. Dispositif selon la revendication 1, dans lequel l'ensemble formant applique est mis en prise sur le support en étant abaissé sur celui-ci.

3. Dispositif selon la revendication 1, et comprenant par ailleurs un moyen servant à solliciter le levier (20) vers sa position de verrouillage.

4. Dispositif selon la revendication 3, dans lequel ledit moyen de sollicitation comprend un ressort (22).

5. Dispositif selon la revendication 3 ou la revendication 4, dans lequel ledit moyen de sollicitation est assisté par la force de gravité.

6. Dispositif selon l'une quelconque des revendications précédentes, dans lequel le support (17) est sous la forme d'un rail allongé.

7. Dispositif selon la revendication 6, dans lequel le rail (17) a une lèvre (19) qui s'étend vers le haut et vers l'extérieur depuis le mur (18).

8. Dispositif selon la revendication 7, dans lequel le bord libre de la lèvre (19) est renversé pour former un profil courbe (24).

9. Dispositif selon la revendication 8, dans lequel le mécanisme de verrouillage (20) est agencé pour venir se mettre en contact avec le support (17) par le biais dudit profil courbe (24).

10. Dispositif selon la revendication 8 ou la revendication 9, dans lequel le bord libre renversé de la lèvre (19) forme ledit rebord orienté vers le bas (25).

11. Dispositif selon l'une quelconque des revendications 8 à 10, dans lequel la hauteur verticale du bord libre renversé de la lèvre (19) est inférieure à la hauteur d'ensemble du support (17).

12. Dispositif selon l'une quelconque des revendications 1 à 11, dans lequel l'axe de la monture pivotante pour le levier (20) est décalé par rapport au point de contact entre l'ensemble formant applique (10) et le support (17).
